Europäisches Patentamt

European Patent Office

Office européen des brevets

(11) Publication number: **0 200 650 B1**

# EUROPEAN PATENT SPECIFICATION

(45) Date of publication of patent specification: **08.12.93**  (51) Int. Cl.⁵: **G01N 33/34**

(21) Application number: **86400930.3**

(22) Date of filing: **28.04.86**

(54) **System and process for measuring the strength of sheet material.**

(30) Priority: **02.05.85 US 730406**

(43) Date of publication of application:
**10.12.86 Bulletin  86/45**

(45) Publication of the grant of the patent:
**08.12.93 Bulletin  93/49**

(84) Designated Contracting States:
**DE FR GB SE**

(56) References cited:
**CH-A- 475 609**
**US-A- 3 823 371**
**US-A- 3 933 035**

(73) Proprietor: **MEASUREX CORPORATION**
**One Results Way**
**Cupertino, California 95014(US)**

(72) Inventor: **Houghton, Paul**
**21050 Bear Creek Road**
**Los Gatos, CA 95030(US)**
Inventor: **Chase, Lee Mac Arthur**
**18400 Overlook Road, no. 60**
**Los Gatos, CA 95030(US)**
Inventor: **Goss, John Douglas**
**6151 Hancock Avenue**
**San Jose, CA 95123(US)**
Inventor: **Norton, Michael Kent**
**18646 Aspesi Court**
**Saratoga, CA 95070(US)**

(74) Representative: **Mongrédien, André et al**
**c/o SOCIETE DE PROTECTION DES INVEN-**
**TIONS**
**25, rue de Ponthieu**
**F-75008 Paris (FR)**

## Description

BACKGROUND OF THE INVENTION

Field of the Invention

The present invention concerns a system for measuring the strength of a sheet such as paper while the sheet is being produced.

State of the Art

In the manufacture of sheet material such as paper it is often important to know the strength of the sheet. For example, strength of paper can be measure in various ways including burst, tensile and pierce strengths. Normally, these strength parameters are determined by various destructive tests in a laboratory. However, on-line determination of paper strength during the manufacture of paper can aid in producing paper which can meet specified strength requirements.

U.S. Patent 4,291,577 teaches a system for measuring the strength of paper as it is being produced. The patent provides for measurement of the velocity of ultrasonic waves through the moving paper sheet. Based upon the velocity, the strength of the paper is determined.

The patent teaches a device having two wheels which are spaced apart from one another and which roll along the moving paper web. The first wheel contains a transducer in the form of a rectangular button mounted on the periphery of the wheel so that as the wheel rotates the button periodically contacts the paper. Each revolution of the wheel, when the transducer contacts the paper, it receives an electrical signal from a signal generator and imparts a mechanical signal to the paper. The second wheel contains a receiving transducer substantially the same as the transmitting transducer, which also is mounted on the periphery of the wheel and occupies a small percentage of the total circumference of the wheel. The receiving transducer contacts the paper once each revolution of the wheel and receives the signal from the transmitter by picking up the ultrasound signal from the paper and converting it to an electrical signal. The system also includes a position detector to monitor the rotational position of the first wheel and to trigger the firing of the ultrasound pulse by the transmitter when the wheel is in a predetermined position. The rotation of the receiving wheel is coordinated with the transmitting wheel so that the receiving transducer is in contact with the paper at the appropriate time to receive the transmitted signal. The signal from the receiving transducer is transmitted to a metering and recording apparatus which measures the velocity of the ultrasonic waves.

The system described in the patent has a number of disadvantages. Since the transmitting transducer and the receiving transducer each contact the paper during only a small percentage of the total rotation of the wheels, the rotation of the wheels must be carefully synchronized and the transmission of the pulse must be carefully timed so that the pulse is received by the transmitter when it is in contact with the paper. Furthermore, since the transmitter and the receiver are only in contact with the paper for a small percentage of the total rotation of the wheels, a substantial portion of the paper is not subject to velocity measurement.

Another system is disclosed in the document US-A-3 933 035. The system disclosed in this patent comprises two driving means which respectively drive a strip at two different speeds. Tension measuring devices are respectively disposed in front of the driving means and they deliver signals which are proportional to the tensions measured. A comparator receives these signals and determines their difference which is proportional to the elastic coefficient of the strip.

The main disadvantage of this system is that it cannot indicate the strength of the strip at different localized areas of this strip.

OBJECTS OF THE INVENTION

An object of the present invention is to provide a system and process for continuously measuring the strength of a moving sheet. A further object is to provide such a system which utilizes the tension and elastic modulus of the sheet and also the velocity of the sheet to determine strength.

To achieve these objects, the present invention provides a system for non-destructively determining the strength of a sheet of material being drawn under tension to travel between a first roll and a second roll, the sheet of material having a width, the system comprising:

a) mounting means located to support a load sensor means for a movement across the width of the sheet while said sheet travels between the first and second rolls;

b) a load sensor attached to the mounting means for contact with the sheet, said load sensor including means for controllably deflecting said sheet across a localized area between the first and second rolls, said load sensor sensing the force exerted by the sheet against the deflecting means at a plurality of localized areas between the first and second rolls and to generate signals corresponding to said force; and

c) computing means for receiving and processing the signals from the load sensor to provide an indication of the strength of the sheet at one localized area relative to the strength at least at one other localized area.

The present invention further provides a process for non-destructively determining the strength of a sheet which is being drawn under tension between a first roll and a second roll comprising the steps of:

a) detecting a deflecting load exerted on the sheet at a plurality of localized areas between the first and second rolls; and,

b) computing the relative strength of the sheet at least at one of said localized areas compared to the strength at least at one other localized areas based upon detected deflecting loads while the sheet is moving between the first and second rolls.

Further objects and advantages of the present invention can be ascertained by reference to the specification and drawings herein which are offered by way of example and not in limitation of the invention which is defined by the claims.

## BRIEF DESCRIPTION OF THE DRAWINGS

Figure 1 is a schematic illustration of the present embodiment.

Figure 2 is an illustration of part of the present embodiment.

Figure 3 is a top view of the part shown in Figure 2.

## DETAILED DESCRIPTION OF THE PREFERRED EMBODIMENTS

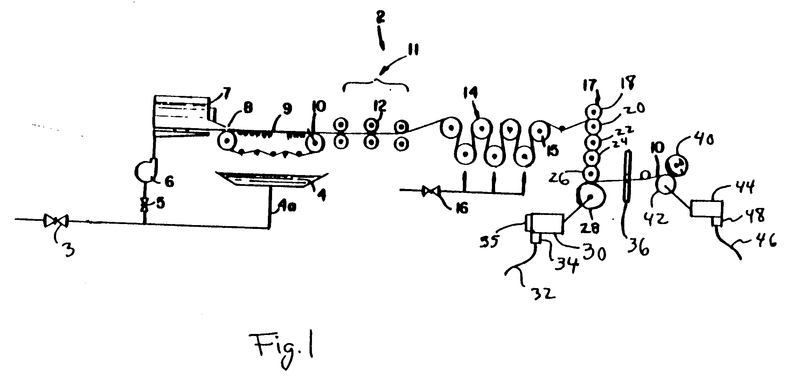

As an aid in understanding the invention, the paper making process is briefly described with reference to Figure 1. A more elaborate description is provided is U.S. Patent 3,757,122 issued September 4, 1973 and entitled "Basis Weight Gauging Apparatus, System and Method Using A Digital Count". With reference to Figure 1, raw stock is supplied to paper machine 2 through a stock valve 3. White water from collection reservoir 4 is drained by a line 4a, mixed with raw stock and supplied through stream valve 5 and fan pump 6 to head box 7. The pulp and white water mixture jets from head box 7 through slice 8 on top of and parallel with wire 9. Water passes through wire 9 and is collected by reservoir 4. However, the majority of fiber is left behind to form a wet sheet of paper 10. After leaving wire 9, sheet 10 is passed through press 11 consisting of a plurality of rollers 12 which removes much of the water from sheet 10. Thereafter the paper sheet passes into a dryer section 14 consisting of a plurality of rollers 15 through which steam is supplied by a steam control valve 16. The steam heats the rollers and consequently evaporates much of the water in the paper sheet so that the paper emerging from dryer section 14 has the desired moisture content. The paper passes from dryer section 14 to a calendar stack 17. The calendar stack 17 includes a plurality of calendar rollers 18, 20, 22, 24, 26 and 28. The bottom most roller, 28, is larger than the other rollers and is driven by a motor 30 which is powered by electricity from line 32. Electricity from line 32 is supplied to the motor 30 through an ammeter 34 which measures the current passing through line 32. A tachometer 35 is coupled to the motor 30 to measure the speed of the motor.

After leaving the calendar rolls 26 and 28, the sheet passes through scanner 36 and is wound onto reel 40. The reel 40 is driven to rotate by a pope roll 42 which in turn is rotated by motor 44. The motor 44 receives electric current through line 46 and ammeter 48. The scanner 36 is conventional and will therefore not be discussed in detail herein. Basically, the scanner 36 includes two heads, one disposed above and the other below the sheet of paper. The scanner 36 also includes a drive system to cause the scanners to reciprocate back and forth across the moving sheet of paper. Further details of a scanner can be ascertained from U.S. Patent 3,621,259 titled "Sheet-Gauging Apparatus".

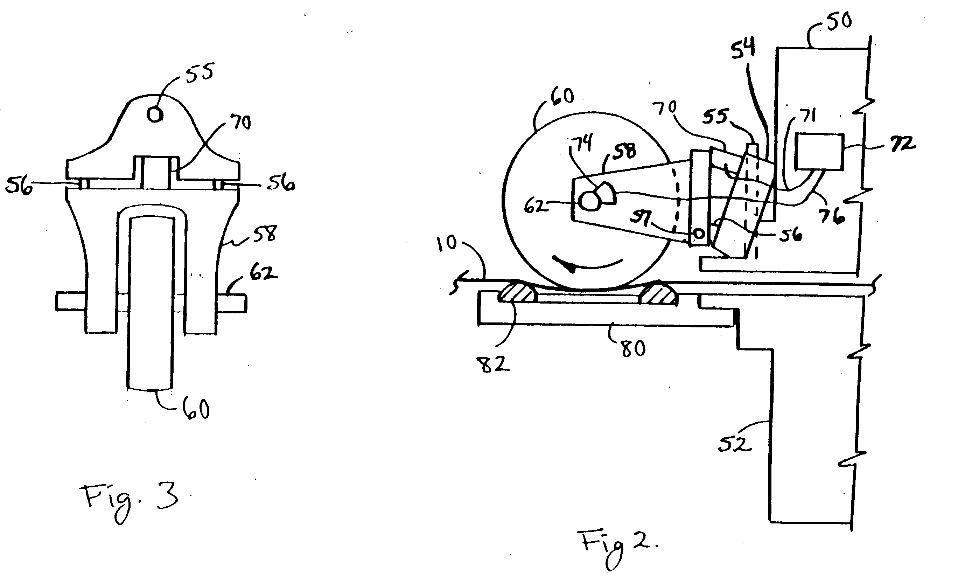

Turning to Figures 2 and 3, there is shown part of an upper gauging head 50 and part of a lower gauging head 52. Both heads 50 and 52 are coupled to the scanner 36 so that they can be moved back and forth across the moving sheet of paper. A support member 54 is coupled to the upper gauging head 50 by pivot 55. The support member includes two brackets 56 mounted at its lower end, and a U-shaped bracket 58 is coupled to the brackets 56 by pivot 57. Thus it can be seen that the U-shaped bracket 58 can rotate horizontally about pivot 55 and vertically about pivot 57.

A wheel 60 is mounted in the U-shaped bracket 57 by an axle 62 on bearings, not shown. Thus, the wheel 60 is free to rotate as indicated by the arrow and also to move upward and downward as the bracket 58 rotates about the pivot 57.

The support member 54 includes an indentation 66 adjacent the upper part of U-shaped bracket 58. A load cell 70 is coupled to the support member 54 in the indentation 66 and also to the bracket 58. The load cell 70 is a conventional device to measure the compressive force on the load cell and generate a signal corresponding to the compressive force. Thus it can be seen that the load cell 70 measures the upward force on the wheel 60. The load cell 70 is coupled by line 71 to transmit signals to a computing system 72 mounted In the upper gauging head 50.

A tachometer 74 is coupled to the bracket 58 and to the axle 62 to measure the rate of rotation of the wheel and generate a signal corresponding thereto. The signals from the tachometer 74 are conveyed to the computing system 72 by a line 76.

A support 80 is coupled to the lower gauging head 52, below the sheet 10, and a sheet support member 82 is coupled to the support 80. The sheet support member 82 is substantially toroidal in shape and has a smooth, rounded upper surface.

In operation, the wheel 60 is mounted above the sheet of paper 10, and the sheet support member 82 is mounted directly below the wheel, and on the opposite side of the sheet of paper. The wheel 60 and sheet support member 82 are positioned so that the sheet 10 is in contact with the upper surface of the support member 82, and the wheel 60 pushes the paper in the center of the support member 82 downwardly a predetermined distance which in practice can be about 0.2 - 0.5 centimeters, and preferably about 0.4 centimeters, below the upper edge of the support member 82 for a support member 82 about 12,7 cm (5 inches) in diameter. Thus increases and decreases in the tension and elastic modulus (i.e. the ratio of stress to strain in the paper) of the sheet 10 impart variable forces on the load cell 70. Higher sheet tension or higher elastic modulus results in higher force on the load cell 70. Also, the wheel 60 is caused to rotate as the sheet 10 moves. The computer 72 receives signals from the load cell 70 corresponding to the tension and elastic modulus in the paper and signals from the tachometer 74 corresponding to the velocity of the paper. The computing system 72 utilizes the data to determine the strength of the sheet of paper as will be discussed in more detail hereinafter.

As the heads 50 and 52 scan across the sheet, the support member 54 and wheel 60 pivot slightly about pivot 55 so that the wheel 60 rolls smoothly on the sheet.

The computing system 72 receives signals from the ammeter 34 which measures the load on the motor 30 driving calendar roller 28. The load on motor 30 is representative of the average tension on the sheet of paper between rollers 15 and the first calendar stack roller 18. The computing system 72 also receives signals from the ammeter 48 which indicate the load on the motor 44 which in turn is representative of the tension on the sheet 10 between the calendar roller 28 and the pope roll 42. The computing system 72 also receives signals from tachometer 35 measuring the speed of motor 30, which is representative of the speed of the sheet of paper 10 as it passes over roller 28.

The computing system 72 utilizes the parameters discussed above to determine the strength of the paper according to the following equation:

$$S = C \left[ \frac{L - T}{(V2 - V1)/V1} \right] + K$$

Where:

S = Strength

C and K = Constants

L = Load at the particular point in the paper measured by the load cell 70 as the system scans across the sheet of paper

T = Average tension of the sheet of paper between roller 15 and roller 18

V2 = Velocity of the sheet measured by the tachometer 74

V1 = Velocity of the sheet as determined from the tachometer 35

In some cases we have found that the calculation of strength can be simplified. In particular, if one desires to know the relative strength of the sheet at one point with respect to the strength at another point, without knowing the absolute value of the strength, it is unnecessary to measure the average tension T or the velocities V1 or V2. Rather, the only parameter necessary is the load, L measured by the load cell 70. We have found that the relative strength of the paper can be determined simply from the relative loads, L, of the paper as measured by our system at various points on the sheet, according to the following equation:

$$\frac{S1}{S2} = \frac{KL1}{L2}$$

Where:

S1 and S2 are strengths at two points

L1 and L2 are loads at the same two points

K is a constant

In some cases we have found that better results can be obtained by determining the strength based upon both the load L and the velocity V2 according to the following equation:

$$S = LV2 \, A + B$$

Where:

S = Strength

L = Load

V2 = Velocity

A and B = Constants

In some cases we have found that better results can be determined by the utilization of the density, $\rho$, of the sheet as well as the load L and velocity V2. In this case the appropriate equation

is:

$$S = \rho LV2\ A2 + B2$$

Where:

The parameters are the same as above, and A2 and B2 are constants.

The density $\rho$ in this case can be determined utilizing the conventionally measured parameters of basis weight and caliper wherein density equals basis weight divided by caliper.

## Claims

1. A system for non-destructively determining the strength of a sheet of material (10) being drawn under tension to travel between a first roll and a second roll (28,42), the sheet of material having a width, the system comprising:

    a) mounting means (50,52) located to support a load sensor for a movement across the width of the sheet while said sheet travels between the first and second rolls;

    b) a load sensor (54-58,60,62,70,80,82) attached to the mounting means for contact with the sheet, said load sensor including means for controllably deflecting said sheet across a localized area between the first and second rolls, said load sensor sensing the force exerted by the sheet against the deflecting means at a plurality of localized areas between the first and second rolls and to generate signals corresponding to said force; and

    c) computing means (72) for receiving and processing the signals from the load sensor to provide an indication of the strength of the sheet at one localized area relative to the strength at least at one other localized area,

2. A system for non-destructively determining the strength of a sheet of material (10) traveling under tension between a first roll and a second roll (28,42), said sheet of material having a width, the system comprising:

    a) mounting means (50,52) located to support a load sensor for movement across the width of the sheet of material while said sheet travels between said first and second rolls;

    b) a load sensor (54-58,60,62,70,80,82) attached to the mounting means for contact with the sheet, said load sensor including means for controllably deflecting said sheet across a localized area between the first and second rolls, said load sensor including means for detecting the force required to accomplish the deflection and for generating an output signal indicative of said force;

    c) velocity detecting means (74) for detecting the velocity of the sheet within said localized area and to generate signals indicative of said velocity; and

    d) computing means (72) for receiving and processing the output signals from the load sensor and the velocity detecting means to provide an output indicative of the strength of the sheet.

3. A system as defined in claim 2, wherein the sheet deflecting means includes a wheel (60) which contacts and deflects the sheet, and said velocity detecting means provides output signals indicating the rate of rotation of said wheel and thereby the velocity of the sheet (10) within said localized area.

4. A system as defined in claim 3, further including density sensing means to sense the density of the sheet (10) and to provide signals corresponding to the sensed density to the computing means (72), the computing means providing an indication of the strength of the sheet based upon signals provided by the load sensor, the density sensing means, and the velocity detecting means.

5. A system as defined in claim 3, including a second velocity detecting means (35) for providing output signals indicative of the velocity of the sheet (10) at a location between said first and second rolls other than within said localized area, the system further including tension sensing means (30,34) mounted to sense the average tension on the sheet and to provide output signals indicative of said average tension, the computing means (72) providing an indication of the strength of the sheet based upon signals provided by the load sensor, the first and second velocity detecting means and the tension sensing means.

6. A process for non-destructively determining the strength of a sheet (10) which is being drawn under tension between a first roll and a second roll (28,42) comprising the steps of:

    a) detecting a deflecting load exerted on the sheet at a plurality of localized areas between the first and second rolls; and,

    b) computing the relative strength of the sheet at least at one of said localized areas compared to the strength at least at one other localized areas based upon detected deflecting loads while the sheet is moving between the first and second rolls.

7. A process according to claim 6, in which the computing step comprises finding values in accordance with the following equation:

$$\frac{S1}{S2} = K\frac{L1}{L2}$$

where:
S1 and S2 =
strengths at two localized areas;
L1 and L2 =
loads at the same two localized areas; and
K =
a constant.

8. A process for non-destructively determining the strength of a sheet (10) of material traveling under tension between a first roll and a second roll (28,42), the process comprising the steps of:
a) controllably deflecting the sheet across a localized area between the first and second rolls;
b) detecting the forces exerted by the sheet when the sheet is deflected at a plurality of localized areas between the first and second rolls;
c) detecting the velocity of the moving sheet at the localized areas; and
d) computing the strength of the sheet at least at one of said localized areas based upon the velocity of the sheet at the localized areas and the deflection forces at said localized areas.

9. A process according to claim 8, wherein the computing step comprises finding values in accordance with the following equation:

$$S = L V2 A + B$$

where:
S =
strength;
A and B =
constants;
L =
the deflection force; and
V2 =
detected velocity of the sheet at the localized areas.

10. A process according to claim 8, further including the step of:
detecting the density of the traveling sheet (10), and wherein the computation of the strength of the sheet is further based on the detected density.

11. A process according to claim 10, in which the computing step comprises finding values in accordance with the following equation:

$$S = \rho L V2A2 + B2$$

where:
S =
strength;
L =
load;
V2 =
velocity of the sheet at the localized areas;
A2 and B2 =
constants; and
$\rho$ =
density.

12. A process according to claim 8, further including the steps of:
detecting the velocity of the sheet (10) at a point between said first and second rolls (28,42) distant from the localized areas; and
detecting the average tension of the traveling sheet, and wherein the computation of the strength of the sheet is further based on said detected distant velocity and said detected average tension.

13. A process according to claim 12, wherein the computing step comprises finding values in accordance with the following equation:

$$S = C\left[\frac{L - T}{(V2 - V1)/V1}\right] + K$$

where:
S =
strength;
C and K =
constants;
L =
detected load;
T =
average tension;
V2 =
velocity of the sheet at said localized areas; and
V1 =
velocity distant from the localized areas.

**Patentansprüche**

1. System zur zerstörungsfreien Bestimmung der Festigkeit einer Materialbahn (10), die sich zwischen einer ersten Walze und einer zweiten Walze (28, 42) bewegend unter Spannung gezogen wird, wobei die Materialbahn eine Breite hat, wobei das System umfaßt:

   a) eine Befestigungseinrichtung (50, 52), die so angeordnet ist, daß sie einen Lastsensor über die Breite der Bahn beweglich trägt, während sich die Bahn zwischen der ersten und der zweiten Walze bewegt;

   b) einen Lastsensor (54-58, 60, 62, 70, 80, 82), der an der Befestigungseinrichtung angebracht ist und mit der Bahn in Kontakt ist, wobei der Lastsensor eine Einrichtung zum gesteuerten Durchbiegen der Bahn über einen örtlich begrenzten Bereich zwischen der ersten und der zweiten Walze enthält, wobei der Lastsensor die Kraft, die durch die Bahn gegen die Durchbiegeeinrichtung ausgeübt wird, an einer Vielzahl von örtlich begrenzten Bereichen zwischen der ersten und der zweiten Walze erfaßt und Signale erzeugt, die der Kraft entsprechen; und

   c) eine Recheneinrichtung (72) zum Empfang und zur Verarbeitung der Signale vom Lastsensor, die eine Anzeige der Festigkeit der Bahn in einem örtlich begrenzten Bereich in bezug auf die Festigkeit in wenigstens einem anderen örtlich begrenzten Bereich erzeugt.

2. System zur zerstörungsfreien Bestimmung der Festigkeit einer Materialbahn (10), die sich unter Spannung zwischen einer ersten und einer zweiten Walze (28, 42) bewegt, wobei die Materialbahn eine Breite hat, wobei das System umfaßt:

   a) eine Befestigungseinrichtung (50, 52), die einen Lastsensor trägt, der sich über die Breite der Materialbahn bewegt, während sich die Bahn zwischen der ersten und der zweiten Walze bewegt;

   b) einen Lastsensor (54-58, 60, 62, 70, 80, 82), der an der Befestigungseinrichtung angebracht ist und mit der Bahn in Kontakt ist, wobei der Lastsensor eine Einrichtung zum gesteuerten Durchbiegen der Bahn über einen örtlich begrenzten Bereich zwischen der ersten und der zweiten Walze enthält, wobei der Lastsensor eine Einrichtung zur Ermittlung der für das Durchbiegen erforderlichen Kraft und zur Erzeugung eines Ausgangssignals, das die Kraft anzeigt, enthält;

   c) eine Geschwindigkeitsermittlungseinrichtung (74) zur Ermittlung der Geschwindigkeit der Bahn innerhalb des örtlich begrenzten Bereichs und zur Erzeugung von Signalen, die die Geschwindigkeit anzeigen; und

   d) eine Recheneinrichtung (72) zum Empfang und zur Verarbeitung der Ausgangssignale vom Lastsensor und der Geschwindigkeitsermittlungseinrichtung, die eine Ausgabe erzeugt, die die Festigkeit der Bahn anzeigt.

3. System nach Anspruch 2, wobei die Bahndurchbiegeeinrichtung ein Rad (60) enthält, das mit der Bahn in Kontakt ist und sie durchbiegt, und die Geschwindigkeitsermittlungseinrichtung Ausgangssignale erzeugt, die die Drehgeschwindigkeit des Rades und damit die Geschwindigkeit der Bahn (10) innerhalb des örtlich begrenzten Bereichs anzeigt.

4. System nach Anspruch 3, das des weiteren eine Dichteerfassungseinrichtung enthält, die die Dichte der Bahn (10) erfaßt und Signale, die der erfaßten Dichte entsprechen, zur Recheneinrichtung (72) leitet, wobei die Recheneinrichtung eine Anzeige der Festigkeit der Bahn auf der Grundlage von Signalen erzeugt, die von dem Lastsensor, von der Dichteerfassungseinrichtung und der Geschwindigkeitsermittlungseinrichtung erzeugt werden.

5. System nach Anspruch 3, das eine zweite Geschwindigkeitsermittlungseinrichtung (35) zur Erzeugung von Ausgangssignalen enthält, die die Geschwindigkeit der Bahn (10) an einer Stelle zwischen der ersten und der zweiten Walze außerhalb des örtlich begrenzten Bereichs anzeigen, wobei das System des weiteren eine Spannungserfassungseinrichtung (30, 34) enthält, die angebracht ist, um die durchschnittliche auf die Bahn wirkende Spannung zu erfassen und Ausgangssignale zu erzeugen, die die durchschnittliche Spannung anzeigen, wobei die Recheneinrichtung (72) eine Anzeige der Festigkeit der Bahn auf der Grundlage der Signale erzeugt, die durch den Lastsensor, die erste und die zweite Geschwindigkeitsermittlungseinrichtung und die Spannungserfassungseinrichtung erzeugt werden.

6. Verfahren zur zerstörungsfreien Bestimmung der Festigkeit einer Bahn (10), die unter Spannung zwischen einer ersten Walze und einer zweiten Walze (28, 42) gezogen wird, das die folgenden Schritte umfaßt:

   a) Ermittlung einer in einer Vielzahl von örtlich begrenzten Bereichen zwischen der ersten und der zweiten Walze auf die Bahn wirkenden Durchbiegelast; und

b) Berechnung der relativen Festigkeit der Bahn in wenigstens einem der örtlich begrenzten Bereiche im Vergleich zur Festigkeit in wenigstens einem anderen örtlich begrenzten Bereich auf der Grundlage ermittelter Durchbiegelasten, während sich die Bahn zwischen der ersten und der zweiten Walze bewegt.

7. Verfahren nach Anspruch 6, wobei der Berechnungsschritt das Auffinden von Werten gemäß der folgenden Gleichung umfaßt:

$$\frac{S1}{S2} = K\frac{L1}{L2}$$

wobei:

S1 und S2 =
Festigkeiten in zwei örtlich begrenzten Bereichen;
L1 und L2 =
Lasten in den gleichen beiden örtlich begrenzten Bereichen; und
K =
eine Konstante.

8. Verfahren zur zerstörungsfreien Bestimmung der Festigkeit einer Materialbahn (10), die sich unter Spannung zwischen einer ersten Walze und einer zweiten Walze (28, 42) bewegt, wobei das Verfahren die folgenden Schritte umfaßt:

a) gesteuertes Durchbiegen der Bahn über einen örtlich begrenzten Bereich zwischen der ersten und der zweiten Walze;

b) Ermittlung der durch die Bahn ausgeübten Kräfte, wenn die Bahn in einer Vielzahl von örtlich begrenzten Bereichen zwischen der ersten und der zweiten Walze durchgebogen wird;

c) Ermittlung der Geschwindigkeit der sich bewegenden Bahn in den örtlich begrenzten Bereichen; und

d) Berechnung der Festigkeit der Bahn in wenigstens einem der örtlich begrenzten Bereiche auf der Grundlage der Geschwindigkeit der Bahn in den örtlich begrenzten Bereichen und der Durchbiegekräfte in den örtlich begrenzten Bereichen.

9. Verfahren nach Anspruch 8, wobei der Berechnungsschritt das Auffinden von Werten gemäß der folgenden Gleichung umfaßt:

S = LV2 A + B

wobei:

S =
Festigkeit;
A und B =
Konstanten;
L =
die Durchbiegekraft; und
V2 =
ermittelte Geschwindigkeit der Bahn in den örtlich begrenzten Bereichen.

10. Verfahren nach Anspruch 8, das des weiteren den folgenden Schritt enthält:
Ermittlung der Dichte der sich bewegenden Bahn (10), und wobei die Berechnung der Festigkeit der Bahn des weiteren auf der erfaßten Dichte beruht.

11. Verfahren nach Anspruch 10, wobei der Berechnungsschritt das Auffinden von Werten gemäß der folgenden Gleichung umfaßt:

S = $\rho$LV2A2 + B2

wobei:

S =
Festigkeit;
L =
Last;
V2 =
Geschwindigkeit der Bahn in den örtlich begrenzten Bereichen;
A2 und B2 =
Konstanten; und
$\rho$ =
Dichte.

12. Verfahren nach Anspruch 8, das des weiteren die folgenden Schritte enthält:
Ermittlung der Geschwindigkeit der Bahn (10) an einem Punkt zwischen der ersten und der zweiten Walze (28, 42), der von den örtlich begrenzten Bereichen entfernt ist; und
Ermittlung der durchschnittlichen Spannung der sich bewegenden Bahn, und wobei die Berechnung der Festigkeit der Bahn des weiteren auf der ermittelten Entfernungsgeschwindigkeit und der ermittelten Durchschnittsspannung beruht.

13. Verfahren nach Anspruch 12, wobei der Berechnungsschritt das Auffinden von Werten gemäß der folgenden Gleichung umfaßt:

$$S = C\left[\frac{L - T}{(V2 - V1)/V1}\right] + K$$

wobei

S =

Festigkeit;

C und K = Konstanten;

L = erfaßte Last;

T = durchschnittliche Spannung;

V2 = Geschwindigkeit der Bahn in den örtlich begrenzten Bereichen; und

V1 = Geschwindigkeit in Entferung von den örtlich begrenzten Bereichen.

**Revendications**

1. Système pour déterminer de façon non destructrice la rigidité d'une feuille de matériau (10) tirée sous tension pour se déplacer entre un premier rouleau et un second rouleau (28,42), la feuille de matériau ayant une largeur, le système comprenant :

   a) des moyens de montage (50,52) situés de manière à supporter un capteur de force pour un mouvement transversal suivant la largeur de la feuille tandis que ladite feuille se déplace entre les premier et second rouleaux;

   b) un capteur de force (54-58,60,62,70,80,82) attaché aux moyens de montage pour venir en contact avec la feuille, ledit capteur de force incluant des moyens pour défléchir ladite feuille de manière contrôlée en travers d'une aire localisée entre les premier et second rouleaux, ledit capteur de force détectant la force exercée par la feuille contre les moyens de déflexion en une pluralité d'aires localisées entre les premier et second rouleaux, et générant des signaux correspondant à ladite force; et

   c) des moyens de calcul (72) pour recevoir et traiter les signaux provenant du capteur de force pour fournir une indication de la rigidité de la feuille en une aire localisée par rapport à la rigidité en au moins une autre aire localisée.

2. Système pour déterminer de façon non destructrice la rigidité d'une feuille de matériau (10) se déplaçant sous tension entre un premier rouleau et un second rouleau (28,42), ladite feuille de matériau ayant une largeur, le système comprenant :

   a) des moyens de montage (50,52) situés de manière à supporter un capteur de force pour un mouvement transversal suivant la largeur de la feuille de matériau tandis que ladite feuille se déplace entre lesdits premier et second rouleaux;

   b) un capteur de force (54-58,60,62,70,80,82) attaché aux moyens de montage pour venir en contact avec la feuille, ledit capteur de force incluant des moyens pour défléchir ladite feuille de manière contrôlée en travers d'une aire localisée entre les premier et second rouleaux, ledit capteur de force incluant des moyens pour détecter la force requise pour accomplir la déflexion et pour générer un signal de sortie indicatif de ladite force;

   c) des moyens de détection de vitesse (74) pour détecter la vitesse de la feuille à l'intérieur de ladite aire localisée et pour générer des signaux indicatifs de ladite vitesse; et

   d) des moyens de calcul (72) pour recevoir et traiter les signaux de sortie provenant du capteur de force et des moyens de détection de vitesse, pour fournir une sortie indicative de la rigidité de la feuille.

3. Système tel que défini dans la revendication 2, dans lequel les moyens de déflexion de la feuille incluent une roue (60) qui vient en contact avec et qui défléchit la feuille, et dans lequel lesdits moyens de détection de vitesse fournissent des signaux de sortie indiquant la vitesse de rotation de ladite roue et par ce moyen la vitesse de la feuille (10) à l'intérieur de ladite aire localisée.

4. Système tel que défini dans la revendication 3, incluant de plus des moyens de détection de densité pour détecter la densité de la feuille (10) et pour fournir des signaux correspondant à la densité détectée aux moyens de calcul (72), les moyens de calcul fournissant une indication de la rigidité de la feuille basée sur les signaux fournis par le capteur de force, par les moyens de détection de densité et par les moyens de détection de vitesse.

5. Système tel que défini dans la revendication 3, incluant des seconds moyens de détection de vitesse (35) pour fournir des signaux de sortie indicatifs de la vitesse de la feuille (10) en un emplacement entre lesdits premier et second rouleaux autre qu'à l'intérieur de ladite aire localisée, le système incluant de plus des moyens de détection de tension (30,34) montés de manière à détecter la tension moyenne sur la feuille et à fournir des signaux de sortie indicatifs de ladite tension moyenne, les moyens de calcul (72) fournissant une indication de la rigidité de la feuille basée sur les signaux fournis par le capteur de force, par les premiers et seconds moyens de détection de vitesse, et par les moyens de détection de tension.

6. Procédé pour déterminer de façon non destructrice la rigidité d'une feuille (10) tirée sous tension entre un premier rouleau et un second rouleau (28,42), comprenant les étapes de :
   a) détection d'une force de déflexion exercée sur la feuille en une pluralité d'aires localisées entre les premier et second rouleaux; et
   b) calcul de la rigidité relative de la feuille en au moins une desdites aires localisées par comparaison avec la rigidité en au moins une autre des aires localisées, basé sur les forces de déflexion détectées tandis que la feuille est en mouvement entre les premier et second rouleaux.

7. Procédé selon la revendication 6, dans lequel l'étape de calcul inclut de trouver des valeurs en accord avec l'équation suivante :

$$\frac{S1}{S2} = K \frac{L1}{L2}$$

où :
   S1 et S2 = rigidités en deux aires localisées;
   L1 et L2 = forces en ces deux aires localisées; et
   K = constante.

8. Procédé pour déterminer de façon non destructrice la rigidité d'une feuille (10) de matériau se déplaçant sous tension entre un premier rouleau et un second rouleau (28,42), le procédé comprenant les étapes de :
   a) déflexion de la feuille de manière contrôlée en travers d'une aire localisée entre les premier et second rouleaux;
   b) détection des forces exercées par la feuille lorsque la feuille est défléchie en une pluralité d'aires localisées entre les premier et second rouleaux;
   c) détection de la vitesse de la feuille en mouvement aux aires localisées; et
   d) calcul de la rigidité de la feuille en au moins une desdites aires localisées, basé sur la vitesse de la feuille aux aires localisées et sur les forces de déflexion auxdites aires localisées.

9. Procédé selon la revendication 8, dans lequel l'étape de calcul inclut de trouver des valeurs en accord avec l'équation suivante :

$$S = L.V2.A + B$$

où :
   S = rigidité;
   A et B = constantes;
   L = force de déflexion; et
   V2 = vitesse détectée de la feuille aux aires localisées.

10. Procédé selon la revendication 8, incluant de plus l'étape de :
    - détection de la densité de la feuille en déplacement (10), et dans lequel le calcul de la rigidité de la feuille est de plus basé sur la densité détectée.

11. Procédé selon la revendication 10, dans lequel l'étape de calcul inclut de trouver des valeurs en accord avec l'équation suivante :

$$S = \rho.L.V2.A2 + B$$

où :
   S = rigidité;
   L = force;
   V2 = vitesse de la feuille aux aires localisées;
   A2 et B2 = constantes; et
   $\rho$ = densité.

12. Procédé selon la revendication 8, incluant de plus les étapes de :
    - détection de la vitesse de la feuille (10) en un point entre lesdits premier et second rouleaux (28,42) distant des aires localisées; et
    - détection de la tension moyenne de la feuille en déplacement, et dans lequel le calcul de la rigidité de la feuille est de plus basé sur ladite vitesse distante détectée et sur ladite tension moyenne détectée.

13. Procédé selon la revendication 12, dans lequel l'étape de calcul inclut de trouver des valeurs en accord avec l'équation suivante :

$$S = C\left[\frac{L - T}{(V2 - V1)/V1}\right] + K$$

où :

S = rigidité;

C et K = constantes;

L = force détectée;

T = tension moyenne;

V2 = vitesse de la feuille auxdites aires localisées; et

V1 = vitesse distante des aires localisées.

Fig. 1

Fig 2.

Fig. 3